Europäisches Patentamt

European Patent Office

Office européen des brevets

(1) Publication number: **0 063 909**
**B2**

(12) **NEW EUROPEAN PATENT SPECIFICATION**

(45) Date of publication of the new patent specification: **12.09.90**

(21) Application number: **82302001.1**

(22) Date of filing: **19.04.82**

(51) Int. Cl.⁵: **C 12 N 9/44,** C 12 P 19/16, C 12 P 19/20, C 12 P 19/22

(54) Debranching enzyme product, preparation and use thereof.

(30) Priority: **20.04.81 US 255952**

(43) Date of publication of application:
**03.11.82 Bulletin 82/44**

(45) Publication of the grant of the patent:
**08.07.87 Bulletin 87/28**

(45) Mention of the opposition decision:
**12.09.90 Bulletin 90/37**

(84) Designated Contracting States:
**AT DE NL**

(56) References cited:
**BE-A- 742 209**
**FR-A-1 553 166**
**FR-A-2 023 548**
**FR-A-2 043 054**
**GB-A-1 466 009**
**US-A-3 827 940**
**US-A-4 011 139**

The file contains technical information submitted after the application was filed and not included in this specification

(73) Proprietor: **NOVO INDUSTRI A/S**
**Novo Allé**
**DK-2880 Bagsvaerd (DK)**

(72) Inventor: **Nielsen, Grethe Camilla**
**Gartnervej 17**
**DK-2630 Tästrup (DK)**
Inventor: **Diers, Ivan Verner**
**Mosegärd Park 85**
**DK-3500 Vaerlose (DK)**
Inventor: **Outtrup, Helle**
**Ravnehusvej 10 C**
**DK-3500 Vaerlose (DK)**
Inventor: **Norman, Barrie Edmund**
**Kielshoj 118**
**DK-3520 Farum (DK)**

(74) Representative: **Brown, John David et al**
**FORRESTER & BOEHMERT Widenmayerstrasse 4/I**
**D-8000 München 22 (DE)**

(56) References cited:
**CHEMICAL ABSTRACTS, vol. 87, no. 15, 10th October 1977, page 252, no. 113656h, Columbus, Ohio, USA K.R. ADAMS et al.: "Extracellular pullulanase synthesis in Bacillus macerans"**

Courier Press, Leamington Spa, England.

# EP  0 063 909  B2

56 References cited:
CHEMICAL ABSTRACTS, vol. 85, no. 17, 25th
October 1976, page 208, no. 118586d,
Columbus, USA Y. TAKASAKI: "Studies on
amylases from Bacillus effective for production
of maltose. Part II. Purifications and enzymic
properties of beta-amylase and pullulanase
from Bacillus cereus var. mycoides"

JOURNAL OF APPLIED BACTERIOLOGY, vol. 46,
1979, pages 291-294, F.J. MORGAN et al.: "A
cultural method for the detection of pullulan -
Degrading enzymes in bacteria and its
application to the genus bacillus"
I. ROITT "Essential Immunology" p. 137-139,
W.H. Axelsen "Manual of quantitatives
Immunoelectrophoresis, chap. 10, 11; Oslo,
1973

## EP 0 063 909 B2

**Description**

This invention is within the field of enzymes for the enzymatic conversion of starch to sugars. More specifically, the present invention relates to a novel debranching enzyme capable of hydrolyzing alpha-1,6-glycosidic bonds in amylopectin and pullulan. This novel enzyme may be classified as a debranching enzyme of the pullulanase type. The invention is also directed towards a process for preparing the novel debranching enzyme and to the use thereof for the conversion of starch into dextrose and/or maltose containing starch hydrolysates, such as dextrose and maltose syrups.

Background of the Invention

During the past decade the all-enzymatic hydrolysis of starch to syrups has gained wide and steadily increasing acceptance within the starch processing industry. On a world-wide basis the present enzymatic production of dextrose syrup from starch is estimated to exceed 3 million tons (calculated as dry substance) per annum compared with about 0.4 million tons ten years ago.

The process generally adopted for enzyme—enzyme hydrolysis of starch encompasses the sequential steps of liquefaction and saccharification, the former being catalysed by an alpha-amylase, such as the thermostable *B. licheniformis* alpha-amylase, e.g. TERMAMYL® supplied by NOVO Industri A/S, Denmark, and the saccharification to dextrose (D-glucose) being effected in the presence of a glucoamylase, usually of fungal origin, such as AMG-150 L, also obtainable from the above named company. Obviously, the dextrose syrup producer aims at obtaining the highest possible yield of dextrose with the least possible expenditure of enzymes and of energy.

The highest dextrose level attainable by the conventional process, starting with a 30—40 percent (by weight) starch suspension and saccharifying at 30 percent dry solids (D.S.), is about 96 percent (by weight) dextrose (96 DX). The reasons why the conventional starch conversion process does not proceed appreciably beyond that limit are essentially twofold.

Firstly, amylopectin (which constitutes about 80% of the majority of industrially important starches, including that of corn or maize), exhibits a branched chain structure in that it contains a significant number of alpha-1,6-glycosidic bonds. Whereas amylopectin is only partially degraded by alpha-amylase because alpha-amylase is practically devoid of alpha-1,6-glucosidase activity, substantial hydrolysis of the branched oligosaccharides including alpha-limit dextrins occurs in the subsequent saccharification step catalyzed by glucoamylase which also hydrolyses alpha-1,6-glycosidic links. However, the latter reaction proceeds at a considerably lower rate than the corresponding hydrolysis of alpha-1,4-bonds, whereby complete saccharification is impeded. Attempts to remedy the situation by adding more glucoamylase collides with a second obstructive feature (apart from incurring higher enzyme costs), namely the ability of glucoamylase also to catalyze dextrose polymerization (the so-called reversionary reaction).

It should be mentioned in passing that an increase of starch conversion from about 96 DX to about 98 DX (which for certain uses of dextrose is regarded as a highly significant improvement) entailing thereby a reduction in the content of non-dextrose contaminants by about 50 percent, can be achieved by employing a relatively high level of glucoamylase combined with a dilution of the substrate to about 15 percent D.S., *vide* U.S. Patent No. 4,017,363. However, the subsequent concentration of such a dextrose solution to the higher conventional dry solids levels in energy consuming

The prior art has suggested employment of glucoamylase and a debranching enzyme simultaneously to obtain a significant increase in dextrose level, the rationale being that debranching enzymes have been shown to efficiently hydrolyse specific types of alpha-1,6-glycosidic bonds occurring in branched chain oligosaccharides, and certain alpha-limit dextrins. In this respect reference is made to U.S. Patent No. 3,897,305 disclosing the combined use of glucoamylase and *Aerobacter aerogenes (Klebsiella pneumoniae)* pullulanase, whereby a significant increase in DX of up to 2 percent can be achieved for syrups containing at least 30 percent D.S. Similar results have been demonstrated for the combined action of glucoamylase and another debranching enzyme, vis. *Pseudomonas amyloderamosa* isoamylase as described in British Patent Application No. 8107287.

However, in the first instance practically no saving of glucoamylase is achieved because the pH optimum of *K. pneumoniae* pullulanase makes it mandatory to conduct the saccharification at a relatively high pH (5,5—6) whereat the activity of glucoamylase is dramatically reduced.

The same problem is not encountered with the isoamylase which has a pH optimum much closer to that of glucoamylase, whereby the dosage of the latter can be substantially reduced (by about 50 percent), simultaneously with the attainment of an increase in DX value of 1—2 percent. However, a serious drawback of the isoamylase process (and actually shared by the known pullulanase process as well) is the heat lability of the debranching enzymes known in the art. This has meant that heretofore no saccharification in the presence of debranching enzyme has been technically feasible above about 55°C, whereas glucoamylase *per se* is adequately stable even at 60°C at which temperature level the risk of microbial contamination of substrates is significantly reduced as compared with lower temperatures.

Obstacles akin to those described hereinbefore have been encountered in the conversion of starch to high maltose syrup by means of beta-amylases. Like the alpha-amylases, beta-amylases are only capable of partially degrading amylopectin, in that hydrolysis thereof stops as an 1,6-alpha-branch point is approached. By combining the action of beta-amylase with that of a debranching enzyme, such as

3

pullulanase or isoamylase, a substantial increase in maltose content can be achieved as disclosed in British Patent No. 1,144,950 and U.S. Patent No. 3,677,896. However, again saccharification temperatures above 55°C are not feasible due to the heat lability of the debranching enzymes, whereby the risk of bacterial contamination is substantially increased.

It is an object of the present invention to obviate the shortcomings of the debranching enzymes known heretofore by furnishing a novel debranching enzyme having a temperature stability comparable to that of glucoamylase, and furthermore, possessing a pH optimum close to that of glucoamylase.

The invention resides in the surprising discovery that a novel debranching enzyme of the pullulanase type having such properties is produced by newly discovered microorganisms as the genus *Bacillus* belonging to the taxonomic group as hereinafter defined.

## Summary of the Invention

According to the first aspect of the present invention there is provided a debranching enzyme product, which comprises a debranching enzyme of the pullulanase type, characterized in that the enzyme product exhibits immunochemical properties at least partially identical to those of the debranching enzyme derived from the *Bacillus* strain NCIB 11607; has an activity optimum measured by incubation for 30 minutes in acetate buffer (0.05 M) at pH 4—5 of at least 60°C, a pH optimum in the range of from 3.5 to 5.5 as determined by incubation for 30 minutes in acetate buffer (0.05 M) at 60°C, and a residual activity after 72 hours at 60°C as measured in a dextrose solution (30% dextrose by weight) at pH 5 of at least 50%.

The debranching enzyme product may be in solid or liquid form and will generally have an activity in the range of from 10 to 350,000 pullulanase units (as hereinafter defined) per g.

In a preferred embodiment of the present invention the activity of the debranching enzyme product is in the range of from 100 to 15,000 pullulanase units per g.

According to a further aspect of the present invention there is provided a process for the preparation of a debranching enzyme product in accordance with the first aspect, which process comprises the cultivation· in a suitable nutrient medium containing carbon and nitrogen sources and inorganic salts of a *Bacillus* strain, followed by recovery of the debranching enzyme product, characterized in that the *Bacillus* strain is selected from NCIB 11607, NCIB 11610, NCIB 11611, NCIB 11636, NCIB 11637 and NCIB 11639, and mutants thereof having the same properties.

In a preferred embodiment of preparing the debranching enzyme product of this invention the *Bacillus acidopullulyticus* strain is selected from the group consisting of NCIB 11607, NCIB 11610, NCIB 11638, and NCIB 11647. Most preferred strains are mutant strains NCIB 11638 and NCIB 11647.

According to another aspect of the present invention there is provided a process for converting starch into syrups containing dextrose and/or maltose, which process comprises conducting the saccharification of starch or starch hydrolysates in the presence of an enzyme system which comprises effective amounts of the debranching enzyme product according to the first aspect of the present invention and a saccharifying enzyme selected from the group consisting of glucoamylase and betamylase; optionally but preferably the saccharification is preceded by a liquefaction step to form a starch hydrolysate.

In a preferred mode of using the debranching enzyme of this invention the dry solids content of the starch hydrolysate is at least 30 percent by weight, the saccharification thereof being conducted in the pH-range of from 3.5 to 5.5 at a temperature in the range of from 55°C to 65°C and preferably not more than 63°C. Preferred dosages of glucoamylase and beta-amylase are in the range of from 0.05 to 0.5 AG units and from 0.005 to 0.3 beta-amylase units, respectively, the preferred dosage of debranching enzyme being in the range of from 0.005 to 5 pullulanase units (as hereinafter defined), per g of dry solids in the starch hydrolysate.

In an additional preferred mode the saccharifying enzyme is glucoamylase, whereby starch is converted into DX dextrose syrup.

## Detailed Descriptions of the Invention

The microorganisms

Isolation: The microorganisms productive of the debranching enzyme of the present invention were selected by means of their ability to grow on a basal medium prepared by aseptic admixture of equal volumes of tryptone solution (1 percent) and a salt solution of the following composition:

| Salt | Percent |
| --- | --- |
| $(NH_4)_2 SO_4$ | 0.04 |
| $MgSO_4, 7H_2O$ | 0.1 |
| $CaCl_2, 2H_2O$ | 0.05 |
| $KH_2PO_4$ | 0.6 |

the pH of the salt solution being adjusted to 3 with sulphuric acid (10N) prior to heat sterilization. The pH of

the final basal medium was 4.8—5.2.

Agar substrates were prepared from the basal medium containing pullulan or amylopectin (0.5 percent), with or without yeast extract (1 percent). Incubation was conducted at 30°C—37°C. Pullulanase activity was detected as clearing zones following precipitation of pullulan by covering the agar plates with acetone. Debranching of amylopectin was detected as zones of hydrolyse exhibiting a strong blue colour with iodine-potassium iodide reagent.

A number of microbial isolates from natural sources, particularly from soil samples, were selected according to the above screening procedure and, when subjected to further tests as hereinafter described, shown to produce the enzyme of the present invention. Examples of such cultures and mutants thereof were deposited with the National Collection of Industrial Bacteria, Torry Research Station, Aberdeen, Scotland and accorded the reference numbers indicated in the following Table 1.

TABLE I

| NCIB No. | Date of deposition | Origin |
|---|---|---|
| 11607 | 8th Sept., 1980 | Soil from zoo, Penang, Malaysia |
| 11610 | 8th Sept., 1080 | Soil collected in Rio de Janeiro |
| 11611 | 8th Sept., 1080 | ibidem |
| 11636 | 17th Febr., 1981 | Soil from citrus plantation, Jamaica |
| 11637 | 17th Febr., 1981 | ibidem |
| 11638 | 17th Febr., 1981 | Mutant of NCIB 11607 |
| 11639 | 17th Febr., 1981 | Soil collected in Hillerod, Denmark |
| 11647 | 7th April, 1981 | Mutant of NCIB 11607 |

Taxonomy

The newly discovered microorganisms of the invention are aerobic, rod shaped and endospore forming bacteria. They therefore belong to the genus *Bacillus*.

Their properties do not fit any of the recognized species of the genus *Bacillus* as described in Bergey's Manual (VIIIth Edition, Williams and Wilkins, Baltimore, 1974), or the monograph: The Genus *Bacillus*, by Gordon, Heynes and Pang, Agriculture Handbook No. 427, US Department of Agriculture, 1973. The present invention therefore classify them as a new taxonomic group to which the name *Bacillus acidopullulyticus* has been accorded.

The diagnosis of this novel taxonomic group is as follows:

Morphology

Vegetative cells: Rods with a diameter of 0.6—1 micron.
Swollen cells of protoplast character are frequently observed and seem to be stable during several hours of submerged fermentation.

Spores: Cylindrical to ellipsoid, central to subterminal, sporangia not swollen. In phase contrast microscopy the spores are difficult to distinguish from unstainable globules which may be present in the protoplasm.
Contrary to these globules the spores are stained by malachite green.

Biochemical reactions:

| | |
|---|---|
| Gram reaction | positive |
| Catalase | positive |
| Aerobic growth | positive |
| Anaerobic growth | negative |
| Growth at 50°C | negative |
| Growth at 30°C—37°C | good |
| Growth in 3.5% NaCl | negative |
| Growth at pH 4.8—5.2 in basal medium (vide supra) containing pullulan as carbon source | good |
| Egg-yolk reaction | negative |

Acid from:

| | | |
|---|---|---|
| | glucose | positive |
| | mannitol | positive |
| | Reduction of nitrate to nitrite | positive |
| | Use of citrate | negative |
| | Use of propionate | negative |
| | Decomposition of tyrosine | negative |
| | Production of a starch debranching pullulanase active at 60°C in the pH-range from 3.5 to 5.5 | positive |
| | VP reaction | variable |
| | Hydrolysis of casein | variable |

Acid from:

| | | |
|---|---|---|
| | xylose | variable |
| | arabinose | variable |

The morphology of the new debranching enzyme producing strains indicates that they belong to the morphological group I of the genus *Bacillus.*

A type culture of the new taxonomic group is NCIB 11607.

Other representative strains of the group are NCIB 11610, 11611, 11636 and 11637, and 11639.

A variety of *Bacillus* species are known producers of pullulan-hydrolysing enzymes (Progress in Industrial Microbiology, vol. 15 (1979), and Morgan F. J., Adams K.R., and Priest F. G.: J. Appl. Bacteriol. *46* p. 291 (1979)). However, none of the known species of *Bacillus* produce a pullulanase retaining its activity at 60°C at pH below 5.0.

Hence, according to an additional aspect of the present invention there is provided a biologically pure culture of a bacterium belonging to the novel taxonomic group of *Bacillus acidopullulyticus* as hereinbefore defined.

Determination of pullulanase activity

One pullulanase unit (PU) is defined as the amount of enzyme which under standard conditions (temperature 60°C and pH 5.0) hydrolyses pullulan at a rate corresponding to the formation of reducing groups equivalent to 1 $\mu$ mole of glucose per minute.

A 4 percent by weight solution (1 ml) of pullulan (supplied by Sigma Chemical Co.) in acetate buffer (0.1 M. pH 5) is preheated for 10 minutes at 60°C followed by addition of a solution (1 ml) of the enzyme dissolved in deionized water at a concentration corresponding to 0.04—0.15 PU per ml. The reaction is stopped after 30 minutes by addition of carbonate buffer, pH 10 (3 ml of 0.5 M). The concentration of reducing groups liberated is then determined by means of the Somogyi-Nelson method (J. Biol. Chem. 153 (1944) 375—80; Ibid. 160 (1945) 61—68).

Preparation of debranching enzyme product

A *Bacillus* strain capable of producing the debranching enzyme of the present invention is usually propagated on a solid substrate prior to its cultivation under aerobic conditions in a suitable fermentation medium. Both media contain assimilable sources of carbon (e.g. glucose for liquid and amylopectin for solid media), a basal salt composition (vide *supra*) comprising e.g. ammonium sulphate as the nitrogen source, together with growth promoting nutrients, such as yeast extract and/or corn steep liquor (liquid medium) or tryptone (solid substrate). The fermentation is typically conducted at slightly elevated temperature, generally in the range of from 30°C to 35°C and at a pH below 6, preferably in the range of 5.0—6.0 and preferably kept approximately constant by automatic means. The enzyme is excreted into the medium.

The ensuing fermentation broth, usually containing from about 0.1 to about 50 PU per ml, may be freed of bacterial cells, debris therefrom together with other solids, for example by centrifugation. The supernatant containing the enzyme may be further clarified, for example by filtration or centrifugation, and then concentrated as required, for example by ultrafiltration of in an evaporator under reduced pressure to give a concentrate which, is desired, may be taken to dryness, for example by lyophilization or spray-drying. Typically, the resulting crude enzyme product exhibits an activity in the range of from 100 to 15,000 PU per gram.

Purification of debranching enzyme

The debranching enzyme of the present invention may be purified from a crude enzyme product, such as the concentrate obtained in Example 2 hereinafter, for example by a combination of cation and anion exchange chromatography.

A column of CM-Sepharose CL-6B was equilibrated with a phosphate-citrate buffer (0.05 M $Na_2HPO_4$ titrated with 0.05 M citric acid to pH 4.5). The enzyme concentrate was diluted with water to the same conductivity as the equilibrating buffer and the pH was adjusted to 4.5. The sample was then applied to the column, whereby the enzyme was completely absorbed. Elution was conducted with the same buffer linearly admixed in a gradient mixer with 0.05 M solution of $Na_2HPO_4$ so as to provide a gradient in pH. The

EP 0 063 909 B2

fractions were analysed for pullulanase activity by the method described hereinbefore and for protein content by the Lowry method (J. Biol. Chem. 193(1951)256), and fractions showing enzyme activity were pooled.

A column of DEAE—Sepharose CL-6B was equilibrated with phosphate-citrate buffer (0.01 M $Na_2HPO_4$ titrated with 0.01 M citric acid to pH 6.5). The pool was diluted to the same conductivity as the equilibrating buffer, adjusted to pH 6.5, and then applied to the column. The completely adsorbed enzyme was eluted with the same buffer admixed with phosphate-citrate buffer (0.15 M $Na_2HPO_4$ titrated to pH 6.5 with 0.15 M citric acid), thereby yielding a linear gradient in buffer concentration. Fractions were collected and analysed as before. The peak fractions showed a specific activity of about 350,000 PU/g. In sodium dodecyl sulphate polyacrylamide gel electrophoresis (S. G. Braun et al., J. Virology vol. 10 (1972) 221) the contents of the fraction with highest activity exhibited in single protein band corresponding to MW about 100,000 Dalton. The protein has a pI of 5.0 as determined by isoelectric focusing on "LKB Ampholine PAG" plates (following the instructions given by LKB-Produkter AB, Sweden).

Enzyme chemical properties

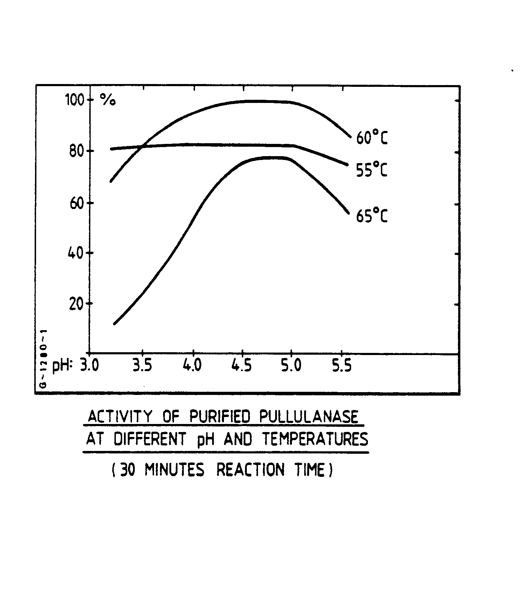

The dependence of the activity of the debranching enzyme of this invention on pH in the range of 3.5—5.5 at different temperature levels was determined by the method for determination of pullulanase activity described above, using a reaction mixture of which pH and temperature was adjusted to predetermined values. Reference is now made to the attached drawing graphically illustrating the relative activity plotted against pH at 55°C, 60°C and 65°C, respectively.

To determine the thermostability of the enzyme in glucose syrup (ca. 30 percent D.S.) a solution of the enzyme product prepared in the following Example 1 was dissolved in dionised water to give a solution containing 3 PU per ml. Aliquots (1 ml) of this solution were mixed with 1 ml of 0.1 M citrate-phosphate buffer and glucose (0.8 g).

After incubation of samples for 3 days at 50°C and 60°C the residual activity was determined by means of the assay for amylopectin debranching activity. A comparative test was conducted with *Pseudomonas* isoamylase (Hayashibara Biochemical Laboratories, Japan, containing 500,000 isoamylase units (IA) per g) diluted to 300 IA per ml.

The results are presented in the following Table II.

TABLE II

| | | Percent residual activity after 3 days | |
| Temperature °C | pH after incubation | Debranching enzyme of present invention | Isoamylase |
| --- | --- | --- | --- |
| 50 | 4.5 | 109 | 75 |
| | 4.9 | 100 | 68 |
| 60 | 4.9 | 89 | 1 |
| | 5.2 | 85 | 3 |

Assay of Amylopectin Debranching Activity

Hydrolysis of the alpha-1,6-bonds of amylopectin causes an increase in the colour intensity (as measured at 610 nm) of the blue iodine-amylopectin complex. This increase is dependent on the amount of alpha-1,6-bonds hydrolysed.

The enzyme solution (1 ml), diluted in deionized water to a concentration corresponding to 1—2 PU and 20—40 IA per mol for debranching enzyme and isoamylase, respectively, is mixed with acetate buffer (1 ml of 0.5 M, pH 4.5) and a 1 percent solution (5 ml) of amylopectin (CPC, SNOWFLAKE 04201 starch). The mixture is incubated for 30 minutes at 50°C. An aliquot (0.5 ml) is mixed with 15 ml of 0.02 N $H_2SO_4$ and 0.5 ml of iodine solution 0.01 M iodine in 0.2 percent potassium iodide).

After 15 minutes at room temperature the optical density at 610 nm is compared with that of a blank run with heat inactivated enzyme.

Immunological properties

Monospecific antiserum was generated by immunizing rabbits with purified debranching enzyme according to the method described by N. H. Axelsen et al., A Manual of Quantitative Immunoelectrophoresis (Oslo 1973) chap. 23. The debranching enzyme was prepared by cultivating the strain NCIB 11638 (cf. Example 1 hereinafter provided) and purified as described above.

The immunogen (0.5 ml of a solution containing 18 PU per ml, corresponding to 0.15 mg of protein per ml) was mixed with Freund's adjuvant (0.5 ml) and injected subcutaneously into rabbits every second week. Antiserum was obtained after a total immunization period of 8 weeks and immunoglobulin was

EP 0 063 909 B2

prepared therefrom as described by N. H. Axelsen et al., *supra*.

Two samples of the crude debranching enzyme product derived from the same strain (NCIB 11638), e.g. as obtained according to Example 1 hereinafter (each sample containing 10 PU per ml), were subjected to crossed immunoelectrophoresis according to the same authors (chap. 3). First dimension: 1 percent agarose in TRIS-boric acid buffer (20 mM, pH 8.6); electrophoresis at 15°C, 8 volts per cm for 2 hours. Second dimension: Same agaraose gel as above containing the immunoglobulin (100 µl); electrophoresis at 15°C, 1 volt per cm for 16 hours.

One plate, stained with Coomassie Brilliant Blue, gave a single peak of immunoprecipitate. The second plate was used for detecting the enzyme by means of an overlayer technique. The plate was covered with gel containing pullulan and agarose (1 percent of each) in maleate buffer (0.1 M, pH 5). After incubation for 2 hours at 50°C the remaining pullulan was precipitated with acetone whereby a clearing zone indicating pullulan hydrolysis became visible. Comparison of the two electropherograms showed that the clearing zone and the stained immunoprecipitate were superimposable, thus demonstrating the monospecificity of the immunoglobulin generated against the debranching enzyme produced by the strain NCIB 11638.

The immunological properties of the debranching enzyme derived from each of the other deposited strains were compared with those of the enzyme from NCIB 11638 by crossed immunoelectrophoresis, using the above prepared immunoglobulin a reference antiserum. The immunograms so obtained demonstrated the identity or partial identity with respect to immunochemical properties of all specimens of debranching enzyme investigated. With respect to the meaning of the terms "immunochemical identity" and "partial immunochemical identity", reference is made to the above cited monograph by N. H. Axelsen et al., chapters 10 and 11, respectively.

The following examples are presented as illustrative embodiments of this invention and are not intended as specific limitations thereof.

Example 1
Preparation of debranching enzyme product from strain NCIB 11638

The strain NCIB 11638 (which is a mutant of NCIB 11607) was propagated on agar slants for 2 days at 37°C. The agar medium was prepared from the basal medium (p. 9) by addition of amylopectin (0.5 percent of Snow Flake waxy starch CPC) and 2 percent Bacto-agar (Difco).

The cells were suspended in sterile water and transferred to a 2 liter laboratory fermentor (Eschweiler, Kiel, West Germany) containing 1 liter of the following medium:

|  | Percent |
|---|---|
| Yeast extract | 0.2 |
| Corn steep liquor (50% dry substance) | 1.0 |
| $MgSO_4 \cdot 7H_2O$ | 0.025 |
| $K_2HPO_4$ | 0.05 |
| $(NH_4)_2SO_4$ | 0.125 |
| $CaCl_2 \cdot 2H_2O$ | 0.025 |
| PLURONIC L 61 dissolved in tap water | 0.1 |

The pH was adjusted to 5.5 prior to autoclaving at 130°C for 30 minutes. Glucose was autoclaved separately and added to a final concentration of 1 percent.

During the cultivation pH was kept at 5.5±0.2 by addition of sulphuric acid (2 percent solution). The temperature was 30.0±0.1°C, and the aeration rate 650 ml per minute (± 10 percent).

After 22 hours of cultivation, substrate exchange was initiated at a dilution rate of 0.03 hour$^{-1}$. After a further 36 hours steady state fermentation conditions were reached at a cell density of about 4 g of dry cells per litre (corresponding to an absorbance of 8 at 450 nm). The pullulanase activity was 7.5 PU per ml.

During 6 days of steady state continuous fermentation, 4,000 ml of culture liquid was collected in a chilled flask. The cells were removed by centrifugation for 30 minutes at 2,300 rpm, and the supernatant, containing 5.1 PU per ml, was concentrated by ultrafiltration through a G-600 membrane using a standard laboratory outfit (supplied by Sartorius). The volume of this concentrate (800 ml) was further reduced in a Büchi rotating evaporator at 45°C. The final concentrate (76 ml) so obtained was frozen and lyophilized, thereby yielding a dry powdered product (6.8 g) having an activity of 1150 PU per g.

Example 2
Preparation of debranching enzyme product from strain NCIB 11647

8

The fermentation was conducted in a stainless steel pilot fermentor (total capacity 550 litres, 70 cm diameter) provided with two 6-blade, 24 cm diameter, turbine agitators and with accessories required for continuous fermentation, including equipment for the maintenance of constant pH and temperature. The fermentor was charged with a medium (300 litres) of the following composition:

| Soy bean meal | 25 | g |
|---|---|---|
| Corn steep liquor (50% dry solids) | 5 | — |
| Potato starch | 8.3 | — |
| KH$_2$PO$_4$ | 1.7 | — |
| (NH$_4$)$_2$SO$_4$ | 1.0 | — |
| Bacterial amylase BAN 120 L | 0.013 | ml |
| Antifoam agent PLURONIC L 61 | 0.233 | — |
| Tap water to | 1 | liter |

The pH of the medium was adjusted to 5.8 with sodium hydroxide solution. The starch was liquefied by raising the temperature gradually from 60°C to 90°C in the course of 50 minutes followed by sterilization at 121°C for 60 minutes.

A culture of the strain NCIB 11647, grown for 4 days in a Fernbach culture flask on the same nutrient agar substrate as used in Example 1, was used for inoculation of 300 litres of medium. The fermentation was conducted at 29°C—31°C, the agitator speed being 100 r.p.m. and the aeration rate 150 standard liters per minute at a pressure of 0.5 bar. Starting at 33.5 hours after inoculation, a sterile medium of the same composition as above and prepared in the same manner was fed continuously to the culture at a rate of 15 liters per hour. When a volume of 375 liters was reached, automatic withdrawal of culture liquid was initiated. Approximately 30 hours after inoculation vigorous growth was observed by microscopy and as indicated by increased generation of CO$_2$.

Collection of culture broth commenced at a culture of 73 hours. The debranching enzyme activity had then increased to above 10 PU per ml. During the period of from 115 hours to 210 hours an approximately steady state was maintained at an enzyme concentration corresponding to between 50 and 76 PU per ml, the pH of the broth varying between 5.4 and 5.9.

All subsequent operations were conducted at pH 5.5—5.7. The fermentation broth collected during 110 hours (1650 liters) was filtered on a rotary vacuum filter (2.5 m$^2$) coated with diatomaceous earth filter aid. The filtrate was concentrated and freed from low molecular weight compounds by ultrafiltration using a DDS (7 m$^2$) module (supplied by DDS, Copenhagen, Denmark) provided with a cellulose acetate membrane, type 600. The final concentrate (70 liters) contained 9.4 percent dry substance.

Further concentration (to a volume of 21 liters), was effected in a rotary vacuum evaporator. An additional pressure filtration, using the same filter aid as above, gave an enzyme concentrate (15.5 kg, containing 28.8 percent dry substance) with an activity of 1500 PU per g.

Example 3
Preparation of debranching enzyme product from strains NCIB 11610, NCIB 11636 and NCIB 11637

The strains were propagated and the fermentations conducted in the same manner as that described in Example 1. Maltose (0.5 percent) was used instead of glucose as the carbon source. Mean values of other parameters of the fermentations will appear from the following Table 1.

| | NCIB 11610 | NCIB 11636 | NCIB 11637 |
|---|---|---|---|
| Dilution rate, hour$^{-1}$ | 0.07 | 0.057 | 0.06 |
| Absorbance at 450 nm | 9.2 | 8.3 | 9.1 |
| Dry weight of bacteria, g/liter | 5.4 | 4.9 | 3.8 |
| Debranching enzyme activity, PU/ml | 0.80 | 0.25 | 0.11 |

The culture broths were collected and the debranching enzyme products were recovered essentially as described in Example 1. Details of the work-up procedure will appear from the following Table 2.

TABLE 2

| Strain NCIB No. | Culture liquid ml | Supernatant | | Lyophilized product | |
|---|---|---|---|---|---|
| | | ml | PU/ml | g | PU/g |
| 11610 | 5316 | 5110 | 0.37 | 7.5 | 69.2 |
| 11636 | 1497 | 1405 | 0.08 | 4.4 | 14.4 |
| 11637 | 4856 | 4790 | 0.04 | 6.0 | 12.2 |

Example 4

Preparation of debranching enzyme product from strain NCIB 11639

Propagation of strain NCIB 11639, incubation and fermentation was conducted as described in Example 1.

The fermentation medium had the following composition:

| | Percent |
|---|---|
| *)Soy bean meal extract | 2.0 |
| Corn steep liquor | 0.5 |
| $MgSO_4, 7H_2O$ | 0.025 |
| $K_2HPO_4$ | 0.1 |
| $(NH_4)_2SO_4$ | 0.05 |
| Amylase-liquefied starch (Dextrose equiv. 11) | 0.5 |
| PLURONIC L 61 dissolved in tap water | 0.1 |

*)Extraction of soy bean meal solubles at pH 4.5 and 50°C for 4 hours. Insoluble material was removed by centrifugation.

The pH was adjusted to 4.0 before autoclaving at 130°C for 60 minutes.

During the cultivation pH was kept at 5.6±0.1 by addition of a sodium hydroxide solution (2 percent). The temperature was 30.0±0.1°C and the aeration rate 320 ml/min at a stirrer speed of 530—565 rpm. The fermentation was run continously at a dilution rate of 0.03—0.05 $hr^{-1}$ for 186 hours when collection of overflowing culture liquid was started. Culture liquid (5100 ml, 0.47 PU/ml) was collected over dry ice for an additional 97 hours under the following conditions:

| | |
|---|---|
| Dilution rate | 0.049±0.008 $hr^-$ |
| $OD_{450}$ | 10.6±1.1 |
| Cell density | 2.9±1.5 g/l |
| Debranching enzyme activity | 0.5±0.1 PU/ml |

The cells were removed as described in Example 1. The supernatant was filtered on a Whatman GFA glass filter (11 cm) and then concentrated to 200 ml on an Amicon DC 2 hollow fibre module provided with a H1P10 membrane. Turbidity was removed on the GFA filter and the filtrate further concentrated on the Amicon module using a 202-DDS 600 membrane (DDS, Copenhagen, Denmark) to yield of final concentrate (30 ml) with an activity of 65 PU/ml. The concentrate was stored deep frozen.

Example 5

100 kg of corn starch (Globe® 03430, CPC) were slurried with tap water containing 100 ppm $Ca^{++}$ and the volume adjusted to 225 liters. The pH was adjusted to 6.3 and 135 g of TERMAMYL® 60 L (NOVO Industri A/B Denmark) were added.

This suspension was continously pumped through a jet cooker (Hydro-Thermal Corp, Milwaukee) where it was heated to 105°C by direct steam injection and maintained at 105°C for five minutes. The liquefied starch suspension was flash-cooled and pumped over into a saccharification tank where it was held for about 1 hour at 95°C.

The pH of the liquefied starch was adjusted to 4.0 to 95°C to stop the reaction and the batch was then spray-dried without purification. The DE of the spray-dried maltodextrin was 6.

Substrates for saccharification were prepared by redissolving suitable amounts of this maltodextrin in deionized water and making up to approximately 30 percent D.S. Aliquots of this substrate were then taken and heated to 60°C and the pH adjusted to 4.8. Different amounts of debranching enzyme were then added together with glucoamylase (Amyloglucosidase Novo 150, 150 AG units/ml). The reaction mixtures were samples at set time intervals and the % dextrose in each sample determined by HPLC. The following results were obtained:

| Debranching enzyme PU units/g D.S. | Glucoamylase AG units/g D.S. | Reaction time (h) | pH | % Dextrose |
|---|---|---|---|---|
| 0 (control) | 0.225 | 72 | 4.1 | 95.8 |
| | 0.225 | 96 | 4.1 | 95.9 |
| 0 (control) | 0.113 | 72 | 4.6 | 93.3 |
| | 0.113 | 96 | 4.3 | 94.4 |
| 0.01 | 0.150 | 96 | 4.3 | 96.1 |
| 0.1 | 0.113 | 96 | 4.4 | 95.9 |
| 0.5 | 0.113 | 96 | 4.4 | 96.7 |
| 1.0 | 0.113 | 72 | 4.6 | 97.0 |
| 2.0 | 0.113 | 72 | 4.7 | 97.3 |
| 4.0 | 0.113 | 72 | 4.7 | 97.4 |

These results indicate that when debranching enzyme is used together with glucoamylase, the same dextrose level can be obtained with half the glucoamylase dosage used in the control when a debranching enzyme dosage of 0.1 PU/g D.S. is used.

If the debranching enzyme dosage is increased the maximum obtainable dextrose level also increases.

Example 6

Aliquots of the substrate prepared as in Example were heated to 55 to 60°C and the pH adjusted to 4.9. An amount of glucoamylase corresponding to 0.113 AG/g D.S. and an amount of debranching enzyme corresponding to 1.2 PU/g D.S. were added. The reaction mixtures were sampled and analyzed as in Example 5.

The following results were obtained:

| Temperature °C | Reaction time (hours) | Dextrose % |
|---|---|---|
| 55 | 24 | 84.5 |
| | 48 | 96.5 |
| | 72 | 97.1 |
| | 96 | 97.3 |
| 60 | 24 | 90.0 |
| | 48 | 96.8 |
| | 72 | 97.0 |
| | 96 | 97.3 |

These results confirm that the debranching enzyme is sufficiently heat stable to be used at 60°C.

Example 7

A spray-dried maltodextrin substrate with a DE of 5 was prepared according to the method described in Example 5.

Aliquots of this substrate were heated to 60, 62.5 and 65°C and the pH adjusted to 4.9. An amount of glucoamylase corresponding to 0.113 AG/g D.S. and an amount of debranching enzyme corresponding to 1 PU/g D.S. were added. Control saccharifications to which an amount of glucoamylase corresponding to 0.225 Ag/g D.S. had been added, but without debranching enzyme, were also included.

The reaction mixtures were sampled and analysed as in Example 1. The dry substance (D.S.) during saccharification was 31 percent.

| Debranching Enzyme PU/g D.S. | Glucoamylase AG/g D.S. | Temperature °C | Reaction time (hours) | Dextrose % |
|---|---|---|---|---|
| 0 | 0.225 | 60 | 24<br>48<br>72<br>96 | 92.1<br>95.3<br>96.0<br>96.0 |
| 1 | 0.113 | 60 | 24<br>48<br>72<br>96 | 88.9<br>96.1<br>96.7<br>97.0 |
| 0 | 0.225 | 62.5 | 24<br>48<br>72<br>96 | 92.1<br>95.1<br>95.8<br>96.1 |
| 1 | 0.113 | 62.5 | 24<br>48<br>72<br>96 | 89.3<br>95.9<br>96.8<br>97.0 |
| 0 | 0.225 | 65 | 24<br>48<br>72<br>96 | 91.4<br>94.1<br>95.2<br>95.4 |
| 1 | 0.113 | 65 | 24<br>48<br>72<br>96 | 82.9<br>89.9<br>92.8<br>93.6 |

These results indicate that saccharification with debranching enzyme and glucoamylase may be carried out at 60—62.5°C. At 65°C the results are inferior, which may be due to glucoamylase instability at that temperature.

Example 8

Aliquots of the substrate prepared as in Example were heated to 60°C and the pH adjusted to 4.5, and 4.8. An amount of glucoamylase corresponding to 0.113 AG/g D.S. and an amount of debranching enzyme corresponding to 1 PU/g D.S. were added. The reaction mixtures were sampled and analyzed as in Example 5. The following results were obtained:

| pH | Reaction time (hours) | % Dextrose |
|---|---|---|
| 4.5 | 24 | 89.6 |
| | 48 | 96.6 |
| | 72 | 96.8 |
| | 96 | 96.9 |
| 4.8 | 24 | 89.0 |
| | 48 | 96.5 |
| | 72 | 96.8 |
| | 96 | 97.0 |

These results indicate that similar results can be obtained when saccharifying at pH 4.5 and 4.8.

Example 9

A 35 percent D.S. (DE 6) substrate was prepared by dissolving a portion of the maltodextrin prepared as described in Example 5 in deionized water. Aliquots of this substrate were taken and heated to 60°C and the pH adjusted to 4.3 and 4.8.

An amount of glucoamylase corresponding to 0.113 AG/g D.S. and an amount of purified debranching enzyme (120 PU/mg protein) corresponding to 1 PU/g D.S. was added. The reaction mixtures were sampled and analyzed as in Example 5. The following results were obtained:

| Reaction time (hours) | pH | % Dextrose |
|---|---|---|
| 0 | 4.3 | — |
| 24 | 4.3 | 89.2 |
| 48 | 4.2 | 96.1 |
| 72 | 4.2 | 96.2 |
| 0 | 4.8 | — |
| 24 | 4.5 | 88.4 |
| 48 | 4.5 | 96.0 |
| 72 | 4.4 | 96.2 |

The results indicate that similar dextrose levels can be obtained when saccharification is started at pH 4.3 to 4.8.

Example 10

Substrates with different dry solids contents were prepared by dissolving 100 g of the DE 6 maltodextrin from Example 5 in different amounts of deionized water and adjusting the pH's to 4.6 at 60°C.

0.133 AG/g D.S. of glucoamylase and 1 PU/g D.S. of debranching enzyme were added. The reaction mixtures were sampled and analyzed as in Example 5. The following results were obtained:

| % D.S. | % Max. Dextrose |
|--------|-----------------|
| 25 | 97.7 |
| 30 | 97.2 |
| 35 | 96.3 |
| 40 | 94.9 |
| 45 | 93.0 |

In a control saccharification without debranching enzyme, the maximum dextrose that could be obtained with this substrate at 60°C, pH 4.5 and 0.225 AG units/g D.S. glucoamylase, and at 30 percent D.S., was 96.3. This means that saccharification may be carried out at a higher solids level when debranching enzyme is used to achieve the same dextrose level so that energy requirements for evaporation will be considerably less.

Example 11

A further batch of spray-dried maltodextrin with a DE of 5.0 was prepared according to the procedure outlined in Example 5.

Substrates for saccharification were prepared by redissolving suitable amounts of this maltodextrin in deionized water and making up to approximately 30 percent. Aliquots of this substrate were then heated to 60°C and the pH adjusted to 5.0.

600 g/ton D.S. beta-amylase (Biozyme M II—Amano Pharmaceutical Co., Ltd, Nagoya, Japan, containing 33.4 beta-amylase units per g) and different amounts of debranching enzyme were added and the reaction mixtures sampled after 72 hours. The percent maltose was determined by HPLC. The following results were obtained.

| Debranching enzyme (PU/g D.S.) | % Dextrose | % Maltose |
|--------------------------------|------------|-----------|
| 0 (control) | 0.3 | 58.7 |
| 0.1 | 0.2 | 63.0 |
| 0.5 | 0.4 | 71.8 |
| 1 | 0.4 | 75.8 |
| 2 | 0.5 | 78.4 |
| 4 | 0.4 | 79.6 |

These results indicate that when debranching enzyme is used in combination with beta-amylase, considerably higher maltose levels can be obtained.

Example 12

114 g of Tapioca starch were slurried with 886 ml of deionized water to which had been added 0.5 g calcium chloride. The slurry was cooked by heating to approximately 100°C and then cooled to 50°C. The pH was adjusted to 5.7 and 0.56 g of Biozyme M II and an amount of debranching enzyme corresponding to 2 PU/g D.S. were added. The pH was kept constant at 5.5. The reaction mixture was sampled and analyzed by HPLC.

| Reaction time (hours) | % Dextrose | % Maltose |
|---|---|---|
| 24 | 0.5 | 77.4 |
| 48 | 0.6 | 84.4 |

This demonstrates that debranching enzyme can be used together with beta-amylase to produce extra-high maltose syrups.

**Claims for the Contracting States: DE NL**

1. A debranching enzyme product, which comprises a debranching enzyme of the pullulanase type, characterized in that the enzyme product exhibits immunochemical properties identical or partially identical to those of the debranching enzyme derived from *Bacillus* strain NCIB 11607; has an activity optimum measured by incubation for 30 minutes in acetate buffer (0.05 M) at pH 4—5 of at least 60°C, a pH optimum in the range of from 3.5 to 5.5 as determined by incubation for 30 minutes in acetate buffer (0.05 M) at 60°C, and a residual activity after 72 hours at 60°C as measured in dextrose solution (30% dextrose by weight) at pH 5 of at least 50% and the enzyme is derivable from a fermentation broth produced by cultivating in a suitable nutrient medium a strain of the taxonomic group herein named *Bacillus acidopullulyticus"*.

2. A debranching enzyme product according to Claim 1, in which the debranching enzyme activity is in the range of from 10—350.000 pullulanase units per g, one pullulanase unit being defined as the amount of enzyme which at 60°C and pH 5.0 hydrolyses pullulan at a rate corresponding to the formation of reducing groups equivalent to 1 micromol of glucose per minute.

3. A debranching enzyme product according to Claim 2, in which the activity is in the range of from 100 to 15,000 pullulanase units per g.

4. A process for the preparation of a debranching enzyme product in accordance with Claim 1, which process comprises the cultivation in a suitable nutrient medium containing carbon and nitrogen sources and inorganic salts of a *Bacillus* strain, followed by recovery of the debranching enzyme product, characterized in the *Bacillus* strain is selected from NCIB 11607, NCIB 11610, NCIB 11611, NCIB 11636, NCIB 11637 and NCIB 11639, and mutants thereof having the same properties.

5. A process according to Claim 4, in which the strain is selected from the mutant group consisting of NCIB 11638 and NCIB 11647.

6. A process for converting starch into syrups containing dextrose and/or maltose, which process comprises conducting the saccharification of starch or starch hydrolysates in the presence of an enzyme system which comprises effective amounts of the debranching enzyme product of Claim 1 and a saccharifying enzyme selected from the group consisting of glucoamylase and betamylase.

7. A process according to Claim 6, further comprising saccharification of a starch hydrolysate of at least 30 percent by weight of dry solids.

8. A process according to Claim 6, in which the saccharification is conducted in the pH-range of from 3.5 to 5.5 at a temperature in the range of from 55° to 65°C.

9. A process according to Claim 6, in which the dosages of glucoamylase and beta-amylase are in the range of from 0.05 to 0.5 AG units and from 0.005 to 0.3 beta-amylase units, respectively, per g of dry solids, one AG unit being defined as the amount of enzyme which hydrolyzes 1 micromol of maltose per minute at 25°C and pH 4.3, and one beta-amylase unit being defined as the number of grams of maltose produced by 1 gram of enzyme from soluble starch (2% w/v) in the course of 30 minutes at 20°C and pH 4.6.

10. A process according to Claim 9, in which the dosage of debranching enzyme is in the range of from 0.005 to 5 pullulanase units per g of dry solids, one pullulanase unit being defined as the amount of enzyme which at 60°C and pH 5.0 hydrolyses pullulan at a rate corresponding to the formation of reducing groups equivalent to 1 micromol of glucose per minute.

11. A biologically pure culture of a *Bacillus* strain selected from NCIB Nos. 11607, 11610, 11611, 11636, 11637, 11638, 11639, 11647.

**Claims for the Contracting State: AT**

1. A process for the preparation of a debranching enzyme product, which process comprises the cultivation in a suitable nutrient medium containing carbon and nitrogen sources and inorganic salts of a *Bacillus* strain, followed by recovery of the debranching enzyme product, characterized in that the recovered debranching enzyme product exhibits immunochemical properties at least partially identical to those of the debranching enzyme derived from the *Bacillus* strain NCIB 11607; has an activity optimum,

EP 0 063 909 B2

measured by incubation for 30 minutes in acetate buffer (0.05 M) at pH 4—5 of at least 60°C, a pH optimum in the range of from 3.5 to 5.5 as determined by incubation for 30 minutes in acetate buffer (0.05 M) at 60°C, and a residual activity after 72 hours at 60°C as measured in dextrose solution (30% D.S. by weight) at pH 5 of at least 50%, the *Bacillus* strain being selected from NCIB 11607, NCIB 11610, NCIB 11639, and mutants thereof having the same properties.

2. A process according to Claim 1, in which the strain is selected from the mutant group consisting of NCIB 11638 and NCIB 11647.

3. A process for converting starch into syrups containing dextrose and/or maltose, which process comprises conducting the saccharification of starch or starch hydrolysates in the presence of an enzyme system which comprises effective amounts of the debranching enzyme product obtainable by the process of Claim 1 or 2 and a saccharifying enzyme selected from the group consisting of glucoamylase and betamylase.

4. A process according to Claim 3, further comprising saccharification of a starch hydrolysate of at least 30 percent by weight of dry solids.

5. A process according to Claim 3, in which the saccharification is conducted in the pH-range of from 3.5 to 5.5 at a temperature in the range of from 55° to 65°C.

6. A process according to Claim 3, in which the dosages of glucoamylase and beta-amylase are in the range of from 0.05 to 0.5 AG units and from 0.005 to 0.3 beta-amylase units, respectively, per g of dry solids, one AG unit being defined as the amount of enzyme which hydrolyzes 1 micromol of maltose per minute at 25°C and pH 4.3, and one beta-amylase unit being defined as the number of grams of maltose produced by 1 gram of enzyme from soluble starch (2% w/v) in the course of 30 minutes at 20°C and pH 4.6.

7. A process according to Claim 6, in which the dosage of debranching enzyme is in the range of from 0.005 to 5 pullulanase units per g of dry solids, one pullulanase unit being defined as the amount of enzyme which at 60°C and pH 5.0 hydrolyses pullulan at a rate corresponding to the formation of reducing groups equivalent to 1 micromol of glucose per minute.

**Patentansprüche für die Vertragsstaaten: DE NL**

1. Zur Abspaltung von Seitenketten befähigtes Enzymprodukt, das ein zur Abspaltung von Seitenketten befähigtes Enzym des Pullulanase-Typs umfaßt, dadurch gekennzeichnet, daß das Enzymprodukt immunochemische Eigenschaften zeigt, die wenigstens teilweise mit denjenigen des zur Abspaltung von Seitenketten befähigten Enzyms identisch sind, das vom *Bacillus*-Stamm NCIB 11607 abgeleitet ist; ein Aktivitätsoptimum von wenigstens 60°C besitzt, gemessen durch 30 minütige Inkubation in Acetatpuffer (0,05 M) bei pH 4 bis 5, ein pH-Optimum im Bereich von 3,5 bis 5,5, wie durch 30 minütige Inkubation in Acetatpuffer (0,05 M) bei 60°C bestimmt, und eine Reaktivität nach 72 Stunden bei 60°C von wenigstens 50% wie in einer Dextroselösung (30 Gew.-% Dextrose) bei pH 5 gemesssen und das Enzym von einer Fermentationsbrühe abgeleitet ist, die hergestellt wird, indem ein Stamm der taxonomen Gruppe, die hierin als *Bacillus acidopullulyticus* bezeichnet wird, in einem geeigneten Nährmedium kultiviert wird.

2. Zur Abspaltung von Seitenketten befähigtes Enzymprodukt nach Anspruch 1, dadurch gekennzeichnet, daß die Aktivität des zur Abspaltung von Seitenketten befähigten Enzyms im Bereich von 10 bis 350 000 Pullulanase-Einheiten pro g liegt, wobei eine Pullulanase-Einheit als die Enzymmenge definiert ist, die bei 60°C und pH 5,0 Pullulan mit einer Geschwindigkeit hydrolysiert, die der Bildung von reduzierenden Gruppen, die 1 Mikromol Glukose äquivalent sind, pro Minute entspricht.

3. Zur Abspaltung von Seitenketten befähigtes Enzymprodukt nach Anspruch 2, dadurch gekennzeichnet, daß die Aktivität im Bereich von 100 bis 15 000 Pullulanase-Einheiten pro g liegt.

4. Verfahren zur Herstellung eines zur Abspaltung von Seitenketten befähigten Enzymprodukts nach Anspruch 1, wobei das Verfahren das Kultivieren eines *Bacillus*-Stammes in einem geeigneten Nährmedium, das Kohlenstoff- und Stickstofflieferanten und anorganische Salze enthält, umfaßt, gefolgt von der Aufbereitung des zur Abspaltung von Seitenketten befähigten Enzymprodukts, dadurch gekennzeichnet, daß der *Bacillus*-Stamm aus NCIB 11607, NCIB 11610, NCIB 11611, NCIB 11636, NCIB 11637 und NCIB 11639 und deren Mutanten mit denselben Eigenschaften ausgewählt wird.

5. Verfahren nach Anspruch 4, dadurch gekennzeichnet, daß der Stamm aus der Mutantengruppe, bestehend aus NCIB 11638 und NCIB 11647, ausgewählt wird.

6. Verfahren zum Umwandeln von Stärke in Sirupe, die Dextrose und/oder Maltose enthalten, wobei das Verfahren das Durchführen der Verzuckerung der Stärke oder der Stärkehydrolysate in Gegenwart eines Enzymsystems umfaßt, das wirksame Mengen des zur Abspaltung von Seitenketten befähigten Enzymproduktes nach Anspruch 1 und ein Verzuckerungsenzym umfaßt, das aus der Gruppe, bestehend aus Glukoamylase und Beta-Amylase, ausgewählt wird.

7. Verfahren nach Anspruch 6, gekennzeichnet durch die Verzuckerung eines Stärkehydrolysats mit wenigstens 30 Gew.-% Trockensubstanz.

8. Verfahren nach Anspruch 6, dadurch gekennzeichnet, daß die Verzuckerung im pH-Bereich von 3,5 bis 5,5 bei einer Temperatur im Bereich von 55°C bis 65°C durchgeführt wird.

9. Verfahren nach Anspruch 6, dadurch gekennzeichnet, daß die Dosierungen von Glukoamylase und Beta-Amylase im Bereich von 0,05 bis 0,5 AG-Einheiten bzw. von 0,005 bis 0,3 Beta-Amylase-Einheiten pro g Trockensubstanz liegen, wobei eine AG-Einheit als die Enzymmenge definiert ist, die bei 25°C und pH 4,3

17

pro Minute ein Mikromol Maltose hydrolysiert, und eine Beta-Amylase-Einheit als die Gramm-menge Maltose definiert ist, die von 1 Gramm Enzym aus löslicher Stärke (2% w/v) im Verlauf von 30 Minuten bei 20°C und pH 4,6 produziert wird.

10. Verfahren nach Anspruch 9, dadurch gekennzeichnet, daß die Dosierung des zur Abspaltung von Seitenketten befähigten Enzyms im Bereich von 0,005 bis 5 Pullulanase-Einheiten pro g Trockensubstanz liegt, wobei eine Pullulanase-Einheit als die Enzymmenge definiert ist, die bei 60°C und pH 5,0 Pullulan mit einer Geschwindigkeit hydrolysiert, die der Bildung von reduzierenden Gruppen, die einem Mikromol Glukose äquivalent sind, pro Minute entspricht.

11. Eine biologisch reine Kultur eines *Bacillus*-Stammes, ausgewählt aus NCIB Nrn. 11607, 11610, 11611, 11636, 11637, 11638, 11639, 11647.

## Patentansprüche für den Vertragsstaat: AT

1. Verfahren zur Herstellung eines zur Abspaltung von Seitenketten befähigten Ezymproduktes, wobei das Verfahren das Kultivieren eines *Bacillus*-Stammes in einem geeigneten Nährmedium, das Kohlenstoff- und Stickstofflieferanten und anorganische Salze enthält, umfaßt, gefolgt von der Aufbereitung des zur Abspaltung von Seitenketten befähigten Enzymproduktes, dadurch gekennzeichnet, daß das aufbereitete, zur Abspaltung von Seitenketten befähigte Enzymprodukt immunochemische Eigenschaften zeigt, die wenigstens teilweise mit denjenigen des zur Abspaltung von Seitenketten befähigten Enzyms identisch sind, das vom *Bacillus*-Stamm NCIB 11607 abgeleitet ist; ein Aktivitätsoptimum von wenigstens 60°C besitzt, gemessen durch 30 minütige Inkubation in Acetatpuffer (0,05 M) bei pH 4 bis 5, ein pH-Optimum im Bereich von 3,5 bis 5,5, wie durch 30 minütige Inkubation in Acetatpuffer (0,05 M) bei 60°C bestimmt, und eine Restaktivität nach 72 Stunden bei 60°C von wenigstens 50%, wie in einer Dextroselösung (30 Gew.-% Trockensubstanz) bei pH 5 gemessen, wobei der *Bacillus*-Stamm aus NCIB 11607, NCIB 11610, NCIB 11639 und deren Mutanten mit denselben Eigenschaften ausgewählt wird.

2. Verfahren nach Anspruch 1, dadurch gekennzeichnet, daß der Stamm aus der Mutantengruppe, bestehend aus NCIB 11638 und NCIB 11647, ausgewählt wird.

3. Verfahren zum Umwandeln von Stärke in Sirupe, die Dextrose und/oder Maltose enthalten, wobei das Verfahren das Durchrühren der Verzuckerung der Stärke oder der Stärkehydrolysate in Gegenwart eines Enzymsystems umfaßt, das wirksame Mengen des zur Abspaltung von Seitenketten befähigten Enzymprodukts, das nach dem Verfahren von Anspruch 1 oder 2 erhalten werden kann, und ein Verzuckerungsenzym umfaßt, das aus der Gruppe, bestehend aus Glukoamylase und Beta-Amylase, ausgewählt wird.

4. Verfahren nach Anspruch 3, gekennzeichnet durch die Verzuckerung eines Stärkehydrolysats mit wenigstens 30 Gew.-% Trockensubstanz.

5. Verfahren nach Anspruch 3, dadurch gekennzeichnet, daß die Verzuckerung im pH-Bereich von 3,5 bis 5,5 bei einer Temperatur im Bereich von 55°C bis 65°C durchgeführt wird.

6. Verfahren nach Anspruch 3, dadurch gekennzeichnet, daß die Dosierungen von Glucoamylase und Beta-Amylase im Bereich von 0,05 bis 0,5 AG-Einheiten bzw. von 0,005 bis 0,3 Beta-Amylase-Einheiten pro g Trockensubstanz liegen, wobei eine AG-Einheit als die Enzymmenge definiert ist, die bei 25°C und pH 4,3 pro Minute ein Mikromol Maltose hydrolysiert, und eine Beta-Amylase-Einheit als die Gramm-menge Maltose definiert ist, die von 1 Gramm Enzym aus löslicher Stärke (2% w/v) im Verlauf von 30 Minuten bei 20°C und pH 4,6 produziert wird.

7. Verfahren nach Anspruch 6, dadurch gekennzeichnet, daß die Dosierung des zur Abspaltung von Seitenketten befähigten Enzyms im Bereich von 0,005 bis 5 Pullulanase-Einheiten pro g Trockensubstanz liegt, wobei eine Pullulanase-Einheit als die Enzymmenge definiert ist, die bei 60°C und pH 5,0 Pullulan mit einer Geschwindigkeit hydrolysiert, die der Bildung von reduzierenden Gruppen, die einem Mikromol glukose äquivalent sind, pro Minute entspricht.

## Revendications pour les Etats contractants: DE NL

1. Produit d'enzyme débranchante, lequel comprend une enzyme débranchante du type pullulanase, caractérisé en ce que le produit d'enzyme présente de propriétés immunochimiques identiques ou partiellement identiques à celles de l'enzyme débranchante dérivée de la souche de *Bacillus* NCIB 11607; en ce qu'elle possède un optimum d'activité mesurée par incubation pendant 30 minutes dans un tampon acétate (0,05 M) à un pH de 4—5 à au moins 60°C, un optimum de pH dans le domaine allant de 3,5 à 5,5 tel que déterminé par incubation pendant 30 minutes dans un tampon acétate (0,05 M) à 60°C, et une activité résiduelle après 72 heures à 60°C, telle que mesurée dans une solution de dextrose (30% de dextrose en poids) à pH 5, d'au moins 50% et l'enzyme peut être dérivée à partir d'un bouillon de fermentation produit par la mise en culture dans un milieu nutritif approprié d'une souche du groupe taxonomique désigné ici *Bacillus acidopullulyticus*.

2. Produit d'enzyme débranchante selon la revendication 1, dans lequel l'activité de l'enzyme débranchante se situe dans le domaine de 10 à 350.000 unités de pullulanase par gramme, une unité de pullulanase étant définie comme la quantité de l'enzyme qui, à 60°C et à pH 5,0, hydrolyse le pullulane à

18

EP 0 063 909 B2

une vitesse correspondant à la formation de groupes réducteurs équivalant à 1 micromole de glucose par minute.

3. Produit d'enzyme débranchante selon la revendication 2, dans lequel l'activité se situe dans le domaine allant de 100 à 15.000 unités de pullulanase par gramme.

4. Procédé pour la preparation d'un produit d'enzyme débranchante selon la revendication 1, lequel procédé comprend la mise en culture dans un milieu nutritif approprié contenant des sources de carbone et d'azote et des sels minéraux d'une souche de *Bacillus*, suivie par la récupération du produit d'enzyme débranchante, caractérisé en ce que la souche de *Bacillus* est choisie parmi la NCIB 11607, la NCIB 11610, la NCIB 11611, la NCIB 11636, la NCIB 11637 et la NCIB 11639, et leurs mutants ayant les mêmes propriétés.

5. Procédé selon la revendication 4, dans lequel la souche est choisie parmi le groupe mutant constitué par la NCIB 11638 et la NCIB 11647.

6. Procédé destiné à convertir l'amidon en sirops contenant du dextrose et/ou du maltose, lequel procédé comprend la réalisation de la saccharification de l'amidon ou d'hydrolysats d'amidon en présence d'un système d'enzyme qui comprend des quantités efficaces du produit d'enzyme débranchante de la revendication 1 et une enzyme saccharifiante choisie dans le groupe constitué par la glucoamylase et la béta-amylase.

7. Procédé selon la revendication 6, comprenant de plus la saccharification d'un hydrolysat d'amidon à au moins 30% en poids de matières solides sèches.

8. Procédé selon la revendication 6, dans lequel la saccharification est conduite dans le domaine de pH allant de 3,5 à 5,5 à une température dans le domaine allant de 55° à 65°C.

9. Procédé selon la revendication 6, dans lequel les doses de glucoamylase et de béta-amylase se situent dans le domaine allant de 0,05 à 0,5 unité d'AG et de 0,005 à 0,3 unités de béta-amylase, respectivement, par gramme de matières solides sèches, une unité d'AG étant définie comme la quantité d'enzyme qui hydrolyse une micromole de maltose par minute à 25°C et à pH 4,3, et une unité de béta-amylase étant définie comme le nombre de grammes de maltose produits par un gramme d'enzyme à partir d'amidon soluble (à 2% en poids/volume) au cours de 30 minutes à 20°C et à pH 4,6.

10. Procédé selon la revendication 9, dans lequel la dose de l'enzyme débranchante se situe dans le domaine allant de 0,005 à 5 unités de pullulanase par gramme de matières solides sèches, une unité de pullulanase étant définie comme la quantité d'enzyme qui, à 60°C et à pH 5,0, hydrolyse le pullulane à une vitesse correspondant à la formation de groupes réducteurs équivalent à 1 micromole de glucose par minute.

11. Culture biologiquement pure d'une souche de *Bacillus* choisie parmi les NCIB No. 11607, 11610, 11611, 11636, 11637, 11638, 11639, 11647.

**Revendications pour l'Etat contractant: AT**

1. Procédé pour la préparation d'un produit d'enzyme débranchante, lequel procédé comprend la mise en culture dans un milieu nutritif approprié contenant des sources de carbone et d'azote et des sels minéraux d'une souche de *Bacillus*, suivie par la récupération du produit d'enzyme débranchante, caractérisé en ce que la produit d'enzyme débranchante récupéré présente des propriétés immunochimiques au moins partiellement identiques à celles de d'enzyme débranchante dérivée de la souche de *Bacillus* NCIB 11607; possède un optimum d'activité mesuré par incubation pendant 30 minutes dans un tampon acétate (0,05 M) à pH 4—5 à au moins 60°C, un optimum de pH dans le domaine allant de 3,5 à 5,5 tel que déterminé par incubation pendant 30 minutes dans un tampon acétate (0,05 M) à 60°C, et une activité résiduelle après 72 heures à 60°C, telle que mesurée dans une solution de dextrose (à 30% de DS en poids) à pH 5 d'au moins 50%, la souche de *Bacillus* étant choisie parmi les NCIB 11607, NCIB 11610, NCIB 11639 et leurs mutants ayant les mêmes propriétés.

2. Procédé selon la revendication 1, dans lequel la souche est choisie dans le groupe mutant consistant en la NCIB 11638 et la NCIB 11647.

3. Procédé destiné à la conversion de l'amidon en sirops contenant du dextrose et/ou du maltose, lequel procédé comprend la réalisation de la sacchrification de l'amidon ou d'hydrolysats d'amidon en présence d'un système d'enzymes qui comprend des quantités efficaces du produit d'enzyme débranchante pouvant être obtenu par le procédé de la revendication 1 ou 2 et une enzyme saccharifiante choisie dans le groupe consistant en la glucoamylase et la béta-amylase.

4. Procédé selon la revendication 3, comprenant de plus la saccharification d'un hydrolysat d'amidon à au moins 30% en poids de matières solides sèches.

5. Procédé selon la revendication 3, dans lequel la saccharification est conduite dans le domaine de pH allant de 3,5 à 5,5 à une température dans le domaine allant de 55° à 65°C.

6. Procédé selon la revendication 3, dans lequel les doses de glucoamylase et de béta-amylase se situent dans le domaine allant de 0,05 à 0,5 unité d'AG et de 0,005 à 0,3 unité de béta-amylase, respectivement, par gramme de matières solides sèches, une unité d'AG étant définie comme la quantité d'enzyme qui hydrolyse une micromole de maltose par minute à 25°C et à pH 4,3, et une unité de béta-amylase étant définie comme le nombre de grammes de maltose produit par une gramme d'enzyme à partir d'amidon soluble (à 2% en poids/volume) au cours de 30 minutes à 20°C et à pH 4,6.

7. Procédé selon la revendication 6, dans lequel la dose d'enzyme débranchante se situe dans le

19

EP 0 063 909 B2

domaine allant de 0,005 à 5 unités de pullulanase par gramme de matières solides sèches, une unité de pullulanase étant définie comme la quantité d'enzyme qui, à 60°C et à pH 5,0 hydrolyse le pullulane à une vitesse correspondant à la formation de groupes réducteurs équivalents à 1 micromole de glucose par minute.

ACTIVITY OF PURIFIED PULLULANASE
AT DIFFERENT pH AND TEMPERATURES
(30 MINUTES REACTION TIME)